# FASCICULE DE BREVET EUROPEEN

(11) **EP 2 683 354 B1**
(45) Date de publication et mention de la délivrance du brevet: **24.05.2017**
(21) Numéro de dépôt: 11810643.4
(22) Date de dépôt: 14.12.2011
(51) Int. Cl.: A61K 8/81, A61Q 19/00, A61K 8/06, C08F 220/06, C08F 220/12, C08F 220/28, C08F 220/58

(54) **PROCÉDÉ D'ÉPAISSISSEMENT D'UNE FORMULATION COSMÉTIQUE À PARTIR D'UNE ÉMULSION ALKALI GONFLABLE D'UN POLYMÈRE À L'AMPS ET RICHE EN ACIDE ACRYLIQUE**
VERDICKUNGSVERFAHREN EINER KOSMETISCHEN ZUBEREITUNG BASIERT AUF EINER ALKALI-QUELLFÄHIGEN EMULSION EINES AMPS POLYMER UND REICH IN ACRYLSÄURE
THICKENING METHOD FOR A COSMETIC COMPOSITION BASED ON AN ALKALI SWELLABLE EMULSION OF AMPS POLYMER AND RICH IN ACRYLIC ACID

(30) Priorité: 07.03.2011 FR 1151812
(43) Date de publication de la demande: 15.01.2014
(73) Titulaire: COATEX, 69730 Genay (FR)
(72) Inventeur: SOUZY, Renaud, F-69300 Caluire et Cuire (FR); SUAU, Jean-Marc, F-69480 Lucenay (FR); KENSICHER, Yves, F-69620 Theize (FR); GUERRET, Olivier, F-46170 Pern (FR)
(86) Numéro de dépôt international: PCT/IB2011/003126
(87) Numéro de publication internationale: WO 2012/120330

(56) Documents cités:
- EP-A2- 1 055 406
- WO-A2-2008/107034
- FR-A1- 2 905 595
- US-A- 4 499 002
- US-A1- 2009 305 934

## Description

La présente invention concerne un procédé d'épaississement d'une composition cosmétique, par mise en oeuvre d'une émulsion directe dans l'eau, alkali gonflable, d'un polymère de type ASE ou HASE, à la fois riche en acide acrylique et doté d'une certaine quantité d'AMPS. C'est la mise en oeuvre de telles émulsions qui permet à la fois de ne pas avoir recours à des tensio-actifs et à des solvants organiques autres que l'eau, et de déclencher le phénomène d'épaississement pour des pH inférieurs à 7 : cette dernière caractéristique est particulièrement avantageuse pour des formulations destinées à être mises en contact avec la peau.

Aujourd'hui, nombre de formulations cosmétiques sont destinées à être appliquées sur la peau : c'est le propre des produits de beauté, de maquillage et de soins du corps. On cherche tout naturellement à épaissir de telles formulations dans une gamme de pH correspondant à celui de la peau, c'est-à-dire à des valeurs comprises entre 5 et 7, et préférentiellement comprises entre 5 et 6,5, très préférentiellement comprises entre 5,5 et 6.

Il existe un certain nombre de solutions techniques à ce problème, qui peuvent être classées en 4 catégories : la mise en oeuvre de polymères de hauts poids moléculaires et sous forme de poudre, la technique dite "retour-acide" qui s'appuie sur des polymères acryliques en émulsion directe de particules polymériques dans l'eau, la mise en oeuvre d'autres polymères toujours sous forme d'émulsions directes, et enfin l'utilisation d'émulsions inverses.

Dans la première catégorie, on peut citer le document EP 1 138 703 A1 qui décrit une composition topique cosmétique, comprenant un polymère de hauts poids moléculaires, à base d'au moins un monomère possédant une fonction acide fort libre copolymérisé avec au moins un monomère estérifié et terminé par un groupe hydrophobe ayant de 8 à 30 atomes de carbone. Le polymère précité est un polymère émulsionnant, sous forme solide ; il peut être dispersé dans l'eau et il permet d'épaissir la composition qui le contient, notamment pour des valeurs de pH voisines de 5.

Toutefois, ces polymères présentent les inconvénients liés à l'emploi d'une poudre : difficultés de transport et de nettoyage, dangerosité du produit en rapport à son caractère pulvérulent, irritant et particulaire. De plus, ces polymères doivent être solubilisés dans le milieu à épaissir, par l'introduction de tensio-actifs. Ces derniers constituent des additifs de formulation supplémentaires qui complexifient la formulation et peuvent interagir avec les tensio-actifs déjà contenus dans ladite formulation, créant parfois des effets indésirables (notamment séparation de phase, formation d'insolubles résiduels).

On connaît aussi la technique dite « retour-acide » (selon l'expression anglophone « back-acid »), telle que décrite dans le document WO 01/76 552. Il s'agit d'un procédé consistant à introduire dans un milieu aqueux un tensio actif et un copolymère acrylique alcali gonflable. Ce dernier conduit à un effet épaississant lorsque ses groupements acides carboxyliques sont neutralisés : il y a alors création d'un réseau tridimensionnel qui conduit à une augmentation de la viscosité de la phase aqueuse. Un tel effet peut être déclenché dans une zone de pH voisine de 6, le rôle du tensio-actif étant de maintenir l'effet épaississant, même lorsqu'on fait diminuer le pH.

Au mécanisme ionique précité, peut s'ajouter un mécanisme associatif, reposant sur la présence d'un monomère hydrophobe : c'est ce que décrit le document WO 03 / 62 288, qui vise aussi à épaissir des formulations à pH acide. Il en va de même du document US 4 529 773 A1. Comme pour la méthode de retour-acide, la présence d'un tensio-actif sous forme d'un produit additionnel est donc nécessaire, entraînant les inconvénients déjà évoqués.

On connaît aussi un certain nombre de documents qui décrivent la mise en oeuvre d'autres polymères en émulsion. A ce titre, le document EP 0 824 914 B1 décrit un polymère contenant un monomère cationique aminé. L'effet épaississant recherché va être obtenu à pH acide via l'ionisation du monomère cationique aminé. Dans le document WO 2004 / 024 779, la cationicité du polymère envisagé est apportée par un monomère vinylique amino substitué. On parvient ici aussi à épaissir un milieu aqueux à pH acide.

Cependant, on connaît bien la toxicité des polymères cationiques sur la faune aquatique : or, on les retrouve malheureusement en fin de cycle de vie dans nos fleuves et nos rivières dans lesquels ils sont déversés par l'intermédiaire du réseau d'eau domestique.

Enfin, on connaît les émulsions inverses et leurs applications comme agents épaississants dans le domaine de la cosmétique, comme divulgué dans les documents WO 2004 063228 A1 et GB 2 422 605 A1. Néanmoins, ces structures requièrent la présence de tensio-actifs et de solvants pour assurer leur stabilité, et on fait alors face aux inconvénients mentionnés plus haut.

Aussi, poursuivant ses recherches en vue d'épaissir des compositions aqueuses, notamment à des pH inférieurs à 7, tout en palliant aux inconvénients des méthodes de l'art antérieur, la Demanderesse a mis au point un procédé d'épaississement employant des structures originales. Il s'agit d'émulsions directes dans l'eau de polymères (méth)acryliques alkali-gonflables, dont 3 caractéristiques essentielles sont :
- l'absence de tensio-actifs et de solvant organique autre que l'eau,
- la présence majoritaire d'acide acrylique vis-à-vis de l'acide méthacrylique,
- et la présence d'une certaine quantité d'un monomère particulier qui est l'acide 2-acrylamido-2-méthylpropane sulfonique (ou AMPS, n° CAS : 40623-75-4).

Ces produits permettent d'épaissir avantageusement une formulation cosmétique, sans pour autant introduire dans celle-ci de nouveaux tensio-actifs, ou des solvants organiques autre que l'eau. De plus, on parvient à déclencher le phénomène d'épaississement dans une zone de pH comprise entre 5 et 7, sans avoir recours à la technique de « retour-acide ».

Or, on ne savait pas réaliser des émulsions alkali-solubles riches en acide acrylique et exemptes de tensio-actifs et de solvants organiques autre que l'eau, avant d'avoir découvert l'importance de l'AMPS dans de telles émulsions. C'est la présence de ce dernier, dans une certaine quantité, qui permet la réalisation de tels produits, aux vertus déjà mentionnées et particulièrement intéressantes dans le domaine de la cosmétique, notamment pour épaissir des formulations à un pH inférieur à 7, notamment compris entre 5 et 6,5, plus préférentiellement entre 5,5 et 6. Ces émulsions ont été décrites pour la première fois dans les Demandes de Brevet Françaises non encore publiées, et déposées sous les numéros FR 10 55080 et FR 10 55077.

La Demanderesse précise que l'expression « émulsion directe d'un polymère dans l'eau » désigne une dispersion stable et homogène de particules de polymère dans l'eau (on ne fait pas référence ici aux émulsions du type huile dans eau ou eau dans huile, qui impliquent l'existence de deux phases distinctes, l'une aqueuse et l'autre huileuse). Quant à l'expression « polymère alkali gonflable », elle signifie que ledit polymère est capable, lorsque le milieu est alcalin, d'incorporer une quantité d'eau telle qu'il y a formation d'un gel et donc augmentation de la viscosité.

Les émulsions visées dans la présente Demande recouvrent 2 grandes familles d'épaississants : ceux de type ASE (Alkali Swellable Emulsion ou émulsion alcali gonflable) et ceux de type HASE (Hydrophobically modified Alkali Swellable Emulsion ou émulsion alkali gonflable et modifiée hydrophobiquement). Les premiers désignent des copolymères de l'acide (méth)acrylique avec un ester non hydrosoluble de ces acides, et les seconds désignent des copolymères à base d'acide (méth)acrylique, d'un ester non hydrosoluble de l'acide (méth)acrylique et d'un monomère disposant de groupements hydrophobes dits « associatifs ». Ces copolymères peuvent en outre être réticulés.

Les mécanismes d'action de ces produits, et notamment le caractère alkali gonflable de ces émulsions et leur capacité à épaissir un milieu aqueux à un pH voisin de la neutralité, ont été décrits dans les documents WO 2007 / 144721 et « Practical guide to associative thickeners » (Proceedings of the Annual Meeting Technical Program of the FSCT, 2000, 78th, 644-702). De manière générale, les épaississants de type ASE et HASE sont fabriqués sous forme d'émulsions directes du polymère alkali gonflable dans l'eau, dont la teneur en produit actif oscille entre 10 % et 45 % de leur poids total.

Le procédé de synthèse correspondant est notamment décrit dans les publications suivantes : « Synthesis of an alkali-swellable emulsion and its effect on the rate of polymer diffusion in poly(vinyl acetate-butyl acrylate) latex films » (Journal of Polymer Science, Part A: Polymer Chemistry, 2005, 43 (22), pp. 5632-5642), « Structural and rheological properties of hydrophobically modified alkali-soluble emulsion solutions » (Journal of Polymer Science, Part B: Polymer Physics, 2002, 40(18), pp. 1985-1994). Il fait également l'objet de nombreuses demandes de brevets (EP 0 089 213 A1, EP 0 646 606 A1, EP 0 979 833 A1 pour les ASE et EP 0 013 836 A1, WO 93 / 2454 A1, US 4 268 641 A1, US 4 421 902 A1, US 3 915 921 A1 pour les HASE).

Aussi, un premier objet de la présente invention consiste en un procédé d'épaississement d'une formulation cosmétique, par mise en contact de ladite formulation avec une émulsion aqueuse directe d'un polymère, puis régulation du pH à une valeur comprise entre 5 et 7, préférentiellement entre 5 et 6,5, très préférentiellement entre 5,5 et 6, caractérisé en ce que ladite émulsion est exempte de tensio-actifs et de solvants organiques autres que l'eau, et en ce que ledit polymère est constitué de, exprimé en % en poids de chacun des monomères :
a) 20 % à 60 % en poids d'acide méthacrylique et d'acide acrylique,
b) 40 % à 80 % en poids d'au moins un monomère choisi parmi l'acrylate d'éthyle, l'acrylate de butyle, le méthacrylate de méthyle et leurs mélanges,
c) 0,05 % à 22 % en poids d'acide 2-acrylamido-2-méthylpropane sulfonique,
d) 0 à 1 % en poids d'au moins un monomère réticulant,
le total a) + b) + c) + d) étant égal à 100 %, ou
a) 20 % à 60 % en poids d'acide méthacrylique et d'acide acrylique,
b) 40 % à 80 % en poids d'au moins un monomère choisi parmi l'acrylate d'éthyle, l'acrylate de butyle, le méthacrylate de méthyle et leurs mélanges,
c) 0,5 % à 25 % en poids d'un monomère contenant un groupement hydrophobe,
d) 0,05 % à 22 % en poids d'acide 2-acrylamido-2-méthylpropane sulfonique,
e) 0 à 1 % en poids d'au moins un monomère réticulant,
le total a) + b) + c) + d) + e) étant égal à 100 %,
le % en poids d'acide acrylique par rapport au poids total des acides acrylique et méthacrylique étant compris entre 50 % et 100 %, préférentiellement entre 80 % et 100 %, très préférentiellement entre 98 % et 100 %, et de manière extrêmement préférentielle ce % est égal à 100 % (il n'y a donc pas d'acide méthacrylique).

Le premier type de composition indiqué plus haut correspond à une émulsion de type ASE riche en acide acrylique, et le second à une émulsion de type HASE riche en acide acrylique.

Ce procédé est aussi caractérisé en ce que le monomère contenant un groupement hydrophobe possède la formule générale R - (OE)ₘ - (OP)ₙ - R', avec :
- m et n désignant des entiers inférieurs ou égaux à 150 dont l'un au moins est non nul,
- OE et OP désignant respectivement l'oxyde d'éthylène et l'oxyde de propylène,
- R désignant une fonction polymérisable, et préférentiellement la fonction méthacrylate ou méthacryluréthane,
- R' désignant un groupement hydrophobe comportant au moins 6 et au plus 36 atomes de carbone.

Ce procédé est aussi caractérisé en ce que le monomère réticulant est choisi parmi le diméthacrylate d'éthylène glycol, le triméthylolpropanetriacrylate, le diallyl phtalate, l'acrylate d'allyle, les maléates d'allyle, le méthylène-bis-acrylamide, le méthylène-bis-méthacrylamide, le tétrallyloxyéthane, les triallylcyanurates, les éthers allyliques et les esters acryliques, méthacryliques et méthacryluréthanes obtenus à partir de polyols.

Ce procédé est aussi caractérisé en ce que l'émulsion aqueuse présente un extrait sec compris entre 10 % et 50 % en poids sec de polymère, par rapport à son poids total.

Ce procédé est aussi caractérisé en ce que l'émulsion présente une taille de particules comprise entre 50 nm et 500 nm.

Ce procédé est aussi caractérisé en ce que le polymère présente une masse molaire moyenne en poids comprise entre 20 000 g/mol et 1 000 000 g/mol.

Ce procédé est enfin caractérisé en ce que ladite formulation cosmétique est choisie parmi les formules contenant au moins un tensio-actif, et notamment parmi celles destinées à être appliquées sur la peau (crèmes de soin, produits de maquillage, moussants, shampoings,...).

Les exemples qui suivent permettront de mieux appréhender l'invention, sans pour autant en limiter la portée.

### EXEMPLES

### Exemple 1

Cet exemple porte sur l'épaississement d'eau à un pH de 5,5.

On a testé 3 émulsions de type HASE selon l'invention, mettant en oeuvre un polymère dont la composition massique est donnée dans le tableau 1.

**Tableau 1**

| Essai n° | AA | AMA | AE | Méthacrylate C₂₂(OE)₂₅ | AMPS |
|---|---|---|---|---|---|
| 1 | 35,7 | 0,6 | 52,5 | 10 | 1,2 |
| 2 | 44,2 | 0,3 | 49,35 | 4,95 | 1,2 |
| 3 | 38,40 | 0,3 | 55,2 | 4,9 | 1,2 |

| | | | | | |
|---|---|---|---|---|---|
| AA : acide acrylique AMA : acide méthacrylique AE : acrylate d'éthyle Méthacrylate C₂₂(OE)₂₅ : monomère de formule générale R - (OE)ₘ - (OP)ₙ - R', avec m = 25, n = 0, OE et OP désignant respectivement l'oxyde d'éthylène et l'oxyde de propylène, R désignant la fonction méthacrylate, R' désignant le groupement alkyle linéaire ayant 22 atomes de carbone AMPS : acide 2-acrylamido-2-méthylpropane sulfonique | | | | | |

On a également mis en oeuvre au cours de l'essai n° 4 un polymère commercialisé par la société Lubrizol™ sous le nom Aqua SF-1.

Pour chaque essai, on a mis en oeuvre 1 % en poids sec du polymère à tester, par rapport au poids total d'eau à épaissir. On a mesuré l'évolution de la viscosité Brookfield™ en mPa.s du gel, mesurée à 100 tours par minute et à 25°C, en fonction du pH qu'on a augmenté par ajout progressif de soude.

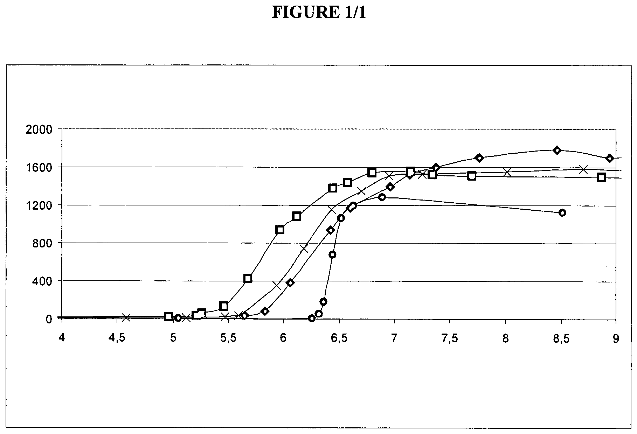

La figure 1/1 fait apparaître cette évolution dans le cas des essais n° 1 (losanges), 2 (carrés), 3 (croix) et 4 (ronds).

Il apparaît clairement que le procédé selon l'invention qui met en oeuvre les émulsions à l'AMPS permet de déclencher le phénomène d'épaississement pour des valeurs de pH bien inférieures qu'avec le polymère commercial.

De plus, le pouvoir épaississant dans la zone de pH comprise entre 5,5 et 6 est plus important pour les polymères de l'invention que pour le polymère de l'art antérieur.

### Exemple 2

Cet exemple porte sur l'épaississement d'eau à un pH de 5,5.

On a testé plusieurs émulsions de type HASE selon l'invention, mettant en oeuvre un polymère dont la composition massique est donnée dans le tableau 1.

**Tableau 2**

| Essai n° | AA | AMA | AE | Méthacrylate TSP(OE)₂₅ | Méthacrylate isoC₁₆(OE)₂₅ | Méthacrylate C₁₈(OE)₃₀ | AMPS |
|---|---|---|---|---|---|---|---|
| 5 | 35 | 1 | 54 | 9 | | | 1 |
| 6 | 45 | 1 | 49 | | 4 | | 1 |
| 7 | 40 | 1 | 49 | | | 9 | 1 |

| | | | | | | | |
|---|---|---|---|---|---|---|---|
| AA : acide acrylique AMA : acide méthacrylique AE : acrylate d'éthyle Méthacrylate TSP(OE)₂₅ : monomère de formule générale R - (OE)ₘ - (OP)ₙ - R', avec m = 25, n = 0, OE et OP désignant respectivement l'oxyde d'éthylène et l'oxyde de propylène, R désignant la fonction méthacrylate, R' désignant le tristyrylphénol (TSP) Méthacrylate isoC₁₆(OE)₂₅ : monomère de formule générale R - (OE)ₘ - (OP)ₙ - R', avec m = 25, n = 0, OE et OP désignant respectivement l'oxyde d'éthylène et l'oxyde de propylène, R désignant la fonction méthacrylate, R' désignant la chaîne alkyle dérivée de l'alcool de Guerbet 2-hexyl 1-decanylol Méthacrylate C₁₈(OE)₃₀ : monomère de formule générale R - (OE)ₘ - (OP)ₙ - R', avec m = 25, n = 0, OE et OP désignant respectivement l'oxyde d'éthylène et l'oxyde de propylène, R désignant la fonction méthacrylate, R' désignant la chaîne alkyle linéaire ayant 18 atomes de carbone. AMPS : acide 2-acrylamido-2-méthylpropane sulfonique. | | | | | | | |

Pour chaque essai, on a mis en oeuvre 1 % en poids sec du polymère à tester, par rapport au poids total d'eau à épaissir. On a mesuré l'évolution de la viscosité Brookfield™ en mPa.s du gel, mesurée à 100 tours par minute et à 25°C, en fonction du pH qu'on a augmenté par ajout progressif de soude.

On a alors repéré le pH pour lequel on atteignait une valeur de viscosité Brookfield égale à 400 mPa.s, sachant que la figure 1 démontre que ce pH est égal à 6,4 environ pour l'essai n° 1 illustrant l'art antérieur.

On obtient pour les essais n° 5,6 et 7 des valeurs de pH respectivement égales à 5,7, 5,8 et 5,9. Ceci démontre bien que le phénomène d'épaississement se déclenche à un pH inférieur à celui observé pour le polymère selon l'art antérieur.

## Revendications

1. Procédé d'épaississement d'une formulation cosmétique, par mise en contact de ladite formulation avec une émulsion aqueuse directe d'un polymère, puis régulation du pH à une valeur comprise entre 5 et 7, préférentiellement entre 5 et 6,5, très préférentiellement entre 5,5 et 6, **caractérisé en ce que** ladite émulsion est exempte de tensio-actifs et de solvants organiques autres que l'eau, et **en ce que** ledit polymère est constitué de, exprimé en % en poids de chacun des monomères :
a) 20 % à 60 % en poids d'acide méthacrylique et d'acide acrylique,
b) 40 % à 80 % en poids d'au moins un monomère choisi parmi l'acrylate d'éthyle, l'acrylate de butyle, le méthacrylate de méthyle et leurs mélanges,
c) 0,05 % à 22 % en poids d'acide 2-acrylamido-2-méthylpropane sulfonique,
d) 0 à 1 % en poids d'au moins un monomère réticulant,
le total a) + b) + c) + d) étant égal à 100 %,
ou
a) 20 % à 60 % en poids d'acide méthacrylique et d'acide acrylique,
b) 40 % à 80 % en poids d'au moins un monomère choisi parmi l'acrylate d'éthyle, l'acrylate de butyle, le méthacrylate de méthyle et leurs mélanges,
c) 0,5 % à 25 % en poids d'un monomère contenant un groupement hydrophobe,
d) 0,05 % à 22 % en poids d'acide 2-acrylamido-2-méthylpropane sulfonique,
e) 0 à 1 % en poids d'au moins un monomère réticulant,
le total a) + b) + c) + d) + e) étant égal à 100 %,
le % en poids d'acide acrylique par rapport au poids total des acides acrylique et méthacrylique étant compris entre 50 % et 100 %, préférentiellement entre 80 % et 100 %, très préférentiellement entre 98 % et 100 %, et de manière extrêmement préférentielle ce % est égal à 100 %.

2. Procédé selon la revendication 1, **caractérisé en ce que** le monomère contenant un groupement hydrophobe possède la formule générale R - (OE)ₘ - (OP)ₙ - R', avec :
- m et n désignant des entiers inférieurs ou égaux à 150 dont l'un au moins est non nul,
- OE et OP désignant respectivement l'oxyde d'éthylène et l'oxyde de propylène,
- R désignant une fonction polymérisable, et préférentiellement la fonction méthacrylate ou méthacryluréthane,
- R' désignant un groupement hydrophobe comportant au moins 6 et au plus 36 atomes de carbone.

3. Procédé selon une des revendications 1 ou 2, **caractérisé en ce que** le monomère réticulant est choisi parmi le diméthacrylate d'éthylène glycol, le triméthylolpropanetriacrylate, le diallyl phtalate, l'acrylate d'allyle, les maléates d'allyle, le méthylène-bis-acrylamide, le méthylène-bis-méthacrylamide, le tétrallyloxyéthane, les triallylcyanurates, les éthers allyliques et les esters acryliques, méthacryliques et méthacryluréthanes obtenus à partir de polyols.

4. Procédé selon une des revendications 1 à 3, **caractérisé en ce que** l'émulsion aqueuse présente un extrait sec compris entre 10 % et 50 % en poids sec de polymère, par rapport à son poids total.

5. Procédé selon une des revendications 1 à 4, **caractérisé en ce que** l'émulsion présente une taille de particules comprise entre 50 nm et 500 nm.

6. Procédé selon une des revendications 1 à 5, **caractérisé en ce que** le polymère présente une masse molaire moyenne en poids comprise entre 20 000 g/mol et 1 000 000 g/mol.

7. Procédé selon une des revendications 1 à 6, **caractérisé en ce que** ladite formulation cosmétique est choisie parmi les formules contenant au moins un tensio-actif, et notamment parmi celles destinées à être appliquées sur la peau.

## Patentansprüche

1. Verfahren zum Verdicken einer kosmetischen Formulierung durch Inberührungbringen der Formulierung mit einer direkten wässrigen Emulsion eines Polymers und anschließende Einstellung des pH-Werts auf einen Wert zwischen 5 und 7, vorzugsweise zwischen 5 und 6,5, ganz besonders bevorzugt zwischen 5,5 und 6, **dadurch gekennzeichnet, dass** die Emulsion frei von Tensiden und organischen Lösungsmitteln außer Wasser ist und dass das Polymer, ausgedrückt in Gew.-% jedes der Monomere, aus Folgendem besteht:
a) 20 bis 60 Gew.-% Methacrylsäure und Acrylsäure,
b) 40 bis 80 Gew.-% mindestens eines Monomers, das aus Ethylacrylat, Butylacrylat, Methylmethacrylat und Mischungen davon ausgewählt ist,
c) 0,05 bis 22 Gew.-% 2-Acrylamido-2-methylpropansulfonsäure,
d) 0 bis 1 Gew.-% mindestens eines Vernetzungsmonomers,
wobei die Summe a) + b) + c) + d) gleich 100% ist,
oder
a) 20 bis 60 Gew.-% Methacrylsäure und Acrylsäure,
b) 40 bis 80 Gew.-% mindestens eines Monomers, das aus Ethylacrylat, Butylacrylat, Methylmethacrylat und Mischungen davon ausgewählt ist,
c) 0,5 bis 25 Gew.-% eines Monomers mit einer hydrophoben Gruppe,
d) 0,05 bis 22 Gew.-% 2-Acrylamido-2-methylpropansulfonsäure,
e) 0 bis 1 Gew.-% mindestens eines Vernetzungsmonomers,
wobei die Summe a) + b) + c) + d) + e) gleich 100% ist,
wobei der Gew.-%-Anteil von Acrylsäure, bezogen auf das Gesamtgewicht von Acryl- und Methacrylsäure, zwischen 50% und 100%, vorzugsweise zwischen 80% und 100% und besonders bevorzugt zwischen 98% und 100% liegt und dieser %-Anteil ganz besonders bevorzugt gleich 100% ist.

2. Verfahren nach Anspruch 1, **dadurch gekennzeichnet, dass** das Monomer mit einer hydrophoben Gruppe die allgemeine Formel R - (OE)ₘ - (OP)ₙ - R' besitzt, wobei:
- m und n für ganze Zahlen kleiner gleich 150 stehen, von denen mindestens eine nicht null ist,
- OE und OP für Ethylenoxid bzw. Propylenoxid stehen,
- R für eine polymerisierbare Funktion und vorzugsweise die Methacrylat- oder Methacrylurethan-Funktion steht,
- R' für eine hydrophobe Gruppe mit mindestens 6 und höchstens 36 Kohlenstoffatomen steht.

3. Verfahren nach einem der Ansprüche 1 oder 2, **dadurch gekennzeichnet, dass** das Vernetzungsmonomer aus Ethylenglykoldimethacrylat, Trimethylolpropantriacrylat, Diallylphtalat, Allylacrylat, Allylmaleaten, Methylenbisacrylamid, Methylenbismethylacrylamid, Tetraallyloxyethan, Triallylcyanuraten, Allylethern und ausgehend von Polyolen erhaltenen Acrylsäure-, Methacrylsäure- und Methacrylurethanestern ausgewählt wird.

4. Verfahren nach einem der Ansprüche 1 bis 3, **dadurch gekennzeichnet, dass** die wässrige Emulsion einen Feststoffgehalt zwischen 10 und 50 Trockengew.-% Polymer, bezogen auf ihr Gesamtgewicht, aufweist.

5. Verfahren nach einem der Ansprüche 1 bis 4, **dadurch gekennzeichnet, dass** die Emulsion eine Teilchengröße zwischen 50 nm und 500 nm aufweist.

6. Verfahren nach einem der Ansprüche 1 bis 5, **dadurch gekennzeichnet, dass** das Polymer eine gewichtsmittlere Molmasse zwischen 20.000 g/mol und 1.000.000 g/mol aufweist.

7. Verfahren nach einem der Ansprüche 1 bis 6, **dadurch gekennzeichnet, dass** die kosmetische Formulierung aus Rezepturen, die mindestens ein Tensid enthalten, und insbesondere aus denjenigen, die für das Aufbringen auf die Haut bestimmt sind, ausgewählt wird.

## Claims

1. Thickening method of a cosmetic formulation, through contact of the said formulation with a direct aqueous emulsion of a polymer, followed by regulation of the pH to a value between 5 and 7, preferentially between 5 and 6.5, very preferentially between 5.5 and 6, **characterised in that** the said emulsion is free from surfactants and organic solvents other than water, and **in that** the said polymer consists, expressed as a % by weight of each of the monomers:
a) 20% to 60% by weight of methacrylic acid and acrylic acid,
b) 40% to 80% by weight of at least one monomer chosen from among ethyl acrylate, butyl acrylate, methyl methacrylate and their blends,
c) 0.05% to 22% by weight of 2-acrylamido-2-methylpropane sulfonic acid,
d) 0 to 1% by weight of at least one cross-linking monomer,
the total a) + b) + c) + d) equals 100%,
or
a) 20% to 60% by weight of methacrylic acid and acrylic acid,
b) 40% to 80% by weight of at least one monomer chosen from among ethyl acrylate, butyl acrylate, methyl methacrylate and their blends,
c) 0.5% to 25% by weight of a monomer containing a hydrophobic group,
d) 0.05% to 22% by weight of 2-acrylamido-2-methylpropane sulfonic acid,
e) 0 to 1% by weight of at least one cross-linking monomer,
the total a) + b) + c) + d) + e) equals 100%,
the % by weight of acrylic acid compared to the total weight of the acrylic and methacrylic acids is between 50% and 100%, preferentially between 80% and 100%, very preferentially between 98% and 100%, and extremely preferentially this % equals 100%.

2. Method according to claim 1, **characterised in that** the monomer containing a hydrophobic group has the general formula R - (EO)ₘ - (PO)ₙ - R', with:
- m and n designating integers of less than or equal to 150, at least one of which is non-zero,
- EO and PO designating respectively ethylene oxide and propylene oxide,
- R designating a polymerisable group, and preferentially the methacrylate or methacrylurethane group,
- R' designating a hydrophobic group having at least 6 and at most 36 carbon atoms.

3. Method according to one of claims 1 or 2, **characterised in that** the cross-linking monomer is chosen from among ethylene glycol dimethacrylate, trimethylolpropanetriacrylate, diallyl phtalate, allyle acrylate, the allyle maleates, methylene-bis-acrylamide, methylene-bis-methacrylamide, tetrallyloxyethane, the triallylcyanurates, the allylic ethers and the acrylic esters, methacrylic and methacrylurethane esters obtained from polyols.

4. Method according to one of claims 1 to 3, **characterised in that** the aqueous emulsion has a solid content of between 10% and 50% by dry weight of polymer, compared to its total weight.

5. Method according to one of claims 1 to 4, **characterised in that** the emulsion has a particle size of between 50 nm and 500 nm.

6. Method according to one of claims 1 to 5, **characterised in that** the polymer has an average molar mass by weight of between 20,000 g/mole and 1,000,000 g/mole.

7. Method according to one of claims 1 to 6, **characterised in that** the said cosmetic formulation is chosen from among the formulations containing at least one surfactant, and in particular from among those intended to be applied to skin.
